**Europäisches Patentamt**

**European Patent Office**

**Office européen des brevets**

(11) Veröffentlichungsnummer: **0 292 928**
**A2**

(12) **EUROPÄISCHE PATENTANMELDUNG**

(21) Anmeldenummer: 88108312.5

(22) Anmeldetag: 25.05.88

(51) Int. Cl.⁴: **G01N 33/53 , G01N 33/68**

(30) Priorität: 25.05.87 CH 2034/87
21.09.87 CH 3649/87

(43) Veröffentlichungstag der Anmeldung:
**30.11.88 Patentblatt 88/48**

(84) Benannte Vertragsstaaten:
**AT BE CH DE ES FR GB GR LI LU NL SE**

(71) Anmelder: **EFTAG ENTSTAUBUNGS- UND FÖRDERTECHNIK AG**
**Ernst-Schüler-Strasse 5**
**CH-2500 Biel 3(CH)**

(72) Erfinder: **Hölter, Heinz, Dipl.-Ing.**
**Beisenstr. 39 - 41**
**D-4390 Gladbeck(DE)**
Erfinder: **Jorde, Wolfgang, Dr. med.**
**Am Spielberg 38**
**D-4050 Mönchengladbach(DE)**
Erfinder: **Schata, Martin, Dr. med.**
**Kesselsbergweg 38**
**D-4000 Düsseldorf 30 (Kaiserswerth)(DE)**

(74) Vertreter: **Spalthoff, Adolf, Dipl.-Ing.**
**Pelmanstrasse 31 Postfach 34 02 20**
**D-4300 Essen 1(DE)**

(54) **Mittel zur Erkennung einer allergischen Reaktion und seine Verwendung in einem Diagnostizierverfahren sowie Vorrichtung zur Verwendung desselben.**

(57) Die Erfindung betrifft ein Mittel zur Erkennung einer allergischen Reaktion eines Organismus auf in diesen eingedrungene Antigene, vorzugsweise auf Umweltantigene, sowie seine Verwendung in einem Diagnostizierverfahren und Vorrichtung zur Verwendung desselben. Um ein Mittel sowie dessen Verwendung in einem Diagnostizierverfahren der eingangs genannten Art und eine Vorrichtung zur Verwendung desselben zu schaffen, welches bzw. welche mit einem vergleichsweise geringen Aufwand bereits in kurzer Zeit eine gesicherte Vorauswahl von allergische Reaktionen zeigenden Personen ermöglicht, wird erfindungsgemäß vorgeschlagen, daß das Mittel aus 0,5 - 800 ngr platelet-activating-factor (paf) in vorzugsweise 0,05 - 0,1 ml eines Lösungsmittels besteht. Die Menge von platelet-activating-factor (paf) beträgt 100 - 400 ngr. Als Lösungsmittel dient eine Coca-Lösung. Die Verwendung des Mittels erfolgt durch Applizierung in die Haut, vorzugsweise die oberste Hautschicht, eines Menschen. Als Vorrichtung zur Verwendung des Diagnosemittels eignet sich besonders ein Stempel, dessen Patrize mit dem Diagnosemittel als Trockensubstanz in der für einen Einmaltest erforderlichen Menge versehen ist. Die Ampulle (1) enthält das Diagnosemittel zur Erkennung einer allergischen Reaktion eines Organismus. Die Ampulle ist mittels eines Stopfens (3) verschlossen wobei dessen die Ampulle (1) verschließende Fläche (4) mit scharfkantigen Vorsprüngen versehen ist. Mit dem Stopfen kann damit z.B. die Haut geritzt werden, um das Diagnosemittel einzusetzen. Das Diagnosemittel kann einfacher und - schneller zur Anwendung gebracht werden; zusätzliche Instrumente sind nicht mehr notwendig.

Fig. 1

**"Mittel zur Erkennung einer allergischen Reaktion und seine Verwendung in einem Diagnostizierverfahren sowie Vorrichtung zur Verwendung desselben"**

Die Erfindung betrifft ein Mittel zur Erkennung einer allergischen Reaktion eines Organismus auf in diesen eingedrungene Antigene, vorzugsweise auf Umweltantigene sowie seine Verwendung in einem Diagnostizierverfahren und Vorrichtung zur Verwendung desselben.

In neuerer Zeit nehmen allergische Reaktionen und daraus resultierende, zum Teil sehr schwere Erkrankungen in unverhältnismäßig starkem Maße zu. Für diese starke Zunahme werden insbesondere in den menschlichen Organismus eingedrungene Umweltantigene verantwortlich gemacht.

Um eine allergische Reaktion eines Menschen zu erkennen ist es bekannt, verschiedenartige Testmethoden anzuwenden. Diese beruhen darauf, daß versucht wird, durch den Einsatz der verschiedensten Mittel Abweichungen des erkanten Organismus gegenüber dem gesunden festzustellen und deren Ursache zu ergründen. Der Nachteil dieser bekannten Testverfahren besteht darin, daß von der ersten Testbehandlung bis zu einer gesicherten Diagnose in der Regel ein Zeitraum von mehreren Wochen erforderlich ist, und außerdem die angewendeten Testverfahren sich als vergleichsweise aufwendig erweisen. Diesen bekannten Testverfahren werden vielfach Menschen unterzogen, bei denen sich im nachhinein herausstellt, daß bei diesen keinerlei allergische Reaktionen vorliegen und daher die mit der Anwendung dieser Testverfahren verbundenen Unannehmlichkeiten für die Testpersonen sowie der dadurch bedingte Aufwand vermeidbar war bzw. waren.

Von diesem Stand der Technik ausgehend liegt der Erfindung die Aufgabe zugrunde, unter Vermeidung vorerwähnter Nachteile ein Mittel sowie dessen Verwendung in einem Diagnostizierverfahren der eingangs genannten Art und eine Vorrichtung zur Verwendung desselben zu schaffen, welches bzw. welche mit einem vergleichsweise geringen Aufwand bereits in kurzer Zeit eine gesicherte Vorauswahl von allergischen Reaktionen zeigenden Personen ermöglicht.

Erfindungsgemäß wird dies dadurch erreicht, daß das Mittel aus 0,5 - 800 ngr platelet-activating-factor (paf) in vorzugsweise 0,05 - 0,1 ml eines Lösungs mittels besteht. Die an sich bekannte Substanz platelet-activating-factor (paf) bewirkt nach ihrem Einbringen in die Haut eines Menschen mittels des als Transportmittels dienenden Lösungsmittels eine Reaktion in Form einer Oedembildung und/oder Hautrötung, wobei diese Reaktion bereits vor Ablauf einer halben Stunde eintritt, so daß eine gesicherte Aussage über das Vorhandensein einer allergischen Reaktion vorliegt.

Vorzugsweise beträgt die Substanz platelet-activating-factor (paf) 100 - 400 ngr.

So wird der Haut einer zu testenden Person beispielsweise eine Menge der Substanz platelet-activating-factor (paf) von 400 ngr in Form einer 0,1 ml-Lösung verabreicht. Es ist aber auch möglich, dieselbe Menge der Substanz mit nur 0,05 ml Lösungsmittel zu verabreichen, um den Lösungsmittelanteil möglichst gering zu halten.

Als Lösungsmittel dient vorteilhaft eine Coca-Lösung. Es kann jedoch auch eine physiologische Kochsalzlösung oder dergleichen Verwendung finden. Die beiden vorerwähnten Lösungsmittel sind an sich bekannt.

Die Erfindung erstreckt sich weiterhin auf die Verwendung des vorerwähnten Diagnosemittels zur Erkennung einer allergischen Reaktion eines Organismus auf in diesen eingedrungene Antigene, vorzugsweise auf Umweltantigene, in einem Diagnostizierverfahren.

Das Diagnosemittel kann mittels einer Injektionsspritze in die Haut injiziert werden. Es ist jedoch auch möglich, einen Tropfen auf die Haut aufzugeben und die Haut unterhalb des Tropfens zu ritzen, so daß der Tropfen in die Haut eindringen kann.

Gegenstand der Erfindung ist weiterhin eine Vorrichtung zur Verwendung des Diagnosemittels, welche aus einem Stempel besteht, dessen Patrize mit dem Diagnosemittel als Trockensubstanz in der für einen Einmaltest erforderlichen Menge versehen ist. Bei der Herstellung wird die Patrize des Stempels in das in flüssiger Form vorliegende Diagnosemittel getaucht, welches nach Aufbringen einem Trocknungsverfahren unterworfen wird, woraufhin der Stempel in an sich bekannter Weise eine sterile Verpackung erhält. Durch Aufdrücken des Stempels wird gleichzeitig die Haut geritzt und das Diagnosemittel übertragen. Hierbei handelt es sich um eine sehr einfach durchzuführende Testmethode, die insbesondere auch bei Reihenuntersuchungen Verwendung finden kann.

Vorteilhaft sind mehrere Stempel zu einer Einheit zusammengefügt, von denen mindestens einer das Diagnosemittel nicht aufweist. Bei Verwendung eines sog. blinden Stempels ergibt sich die Möglichkeit einer Negativkontrolle um auszuschließen, daß die Testperson auf das reine Ritzen der Haut eine Reaktion entwickelt, die zu Fehlschlüssen Anlaß geben könnte.

Nach einem weiteren Vorschlage der Erfindung kann die Vorrichtung zur Verwendung des Diagnosemittels auch aus einer Ampulle bestehen, welche das Diagnosemittel als Flüssigkeit in der für einen Einmaltest erforderlichen Menge enthält. Deren zugeschmolzener Halsbereich ist derart mit einer Sollbruchstelle versehen, daß nach Abbrechen des die Sollbruchstelle überragenden Teils des Halsbereiches im Bereich des verbleibenden Teils der Sollbruchstelle eine scharfkantige Schneide vorhanden ist. Auch mit Hilfe dieser Vorrichtung gestaltet sich die Durchführung des Tests als vergleichsweise einfach und schnell, da lediglich der die Sollbruchstelle überragende Teil des Halsbereiches abgebrochen zu werden braucht und mit der verbleibenden scharfen Kante die Haut geritzt und der Inhalt der Ampulle auf die geritzte Haut übergeben wird.

Gemäß einem weiteren Vorschlag der Erfindung wird zur einfachen und schnellen Anwendung des Diagnosemittels eine Vorrichtung vorgeschlagen, welche eine Ampulle aufweist, welche das Diagnosemittel als Flüssigkeit in der für einen Einmaltest erforderlichen Menge enthält und einendig mittels eines Stopfens od.dgl. dicht verschlossen ist, dessen dem Diagnosemittel zugewandte Stirnfläche mit einem oder mehreren nadelartigen und/oder scharfkantigen Vorsprüngen od.dgl. ausgerüstet ist.

Zur Durchführung eines Tests wird die Ampulle auf den Kopf gestellt, so daß die nadelartigen oder scharfkantigen Vorsprünge mit dem flüssigen Diagnosemittel benetzt sind. Durch Aufdrücken des Stopfens od.dgl. wird die Haut durchstochen und/oder geritzt und dabei das anhaftende Diagnosemittel auf die Testperson übertragen, woraufhin der Inhalt der Ampulle auf die geritzte Haut übergeben wird.

Ein Ausführungsbeispiel der Erfindung ist an Hand der Zeichnung näher erläutert, und zwar zeigt:

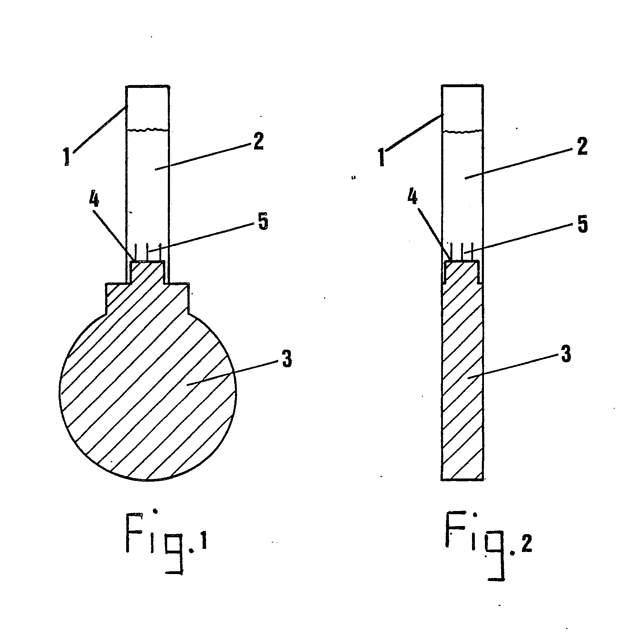

Figur 1 eine Ansicht der Vorrichtung und
Figur 2 eine Seitenansicht der Figur 1.

Mit 1 ist die Ampulle bezeichnet, welche das flüssige Diagnosemittel 2 enthält. Einendig ist die Ampulle 1 mittels des Stopfens 3 dicht verschlossen, so daß das in der Ampulle 1 enthaltene Diagnosemittel 2 nicht unbeabsichtigt aus der Ampulle 1 austreten kann. Die dem Diagnosemittel 2 zugewandte Stirnfläche 4 des Stopfens 3 ist mit mehreren nadelartigen und/oder scharfkantigen Vorsprüngen 5 ausgerüstet.

Das der Ampulle 1 abgewandte Ende des Stopfens 3 ist als Handhabe, beispielsweise als Griff, Stiel od.dgl., ausgeführt. Im dargestellten Ausführungsfalle ist ein plattenförmiger Stiel vorgesehen.

Zur Durchführung eines Tests wir die Vorrichtung so gehalten, daß das flüssige Diagnosemittel die Stirnfläche 4 des Stopfens 3 und damit dessen Vorsprünge 5 benetzt. Nach Zurückschwenken der Ampulle 1 wird der Stopfen herausgezogen und die mit dem Diagnosemittel benetzten Vorsprünge 5 in die Haut einer Testperson eingedrückt, woraufhin der Rest der Flüssigkeit auf die geritzte Hautoberfläche gegeben wird

**Ansprüche**

1. Mittel zur Erkennung einer allergischen Reaktion eines Organismus auf in diesen eingedrungene Antigene, vorzugsweise auf Umweltantigene, dadurch gekennzeichnet, daß dieses aus 0,5 - 800 ngr platelet-activating-factor (paf) in vorzugsweise 0,05 - 0,1 ml eines Lösungsmittels besteht.

2. Mittel nach Anspruch 1, dadurch gekennzeichnet, daß die Menge von platelet-activating-factor (paf) 100 - 400 ngr beträgt.

3. Mittel nach Anspruch 1 oder 2, dadurch gekennzeichnet, daß als Lösungsmittel eine Coca-Lösung, eine physiologische Kochsalzlösung oder dergleichen dient.

4. Verwendung des Mittels nach Anspruch 1 oder einem der vorhergehenden zur Erkennung einer allergischen Reaktion eines Organismus durch Applizierung in die Haut, vorzugsweise die oberste Hautschicht, eines Menschen.

5. Vorrichtung zur Verwendung des Diagnosemittels nach Anspruch 1 oder einem der vorhergehenden, dadurch gekennzeichnet, daß diese aus einem Stempel besteht, dessen Patrize mit dem Diagnosemittel als Trockensubstanz in der für einen Einmaltest erforderlichen Menge versehen ist.

6. Vorrichtung nach Anspruch 5, dadurch gekennzeichnet, daß mehrere Stempel zu einer Verpackungseinheit zusammengefügt sind, von denen mindestens einer das Diagnosemittel nicht aufweist.

7. Vorrichtung zur Verwendung des Diagnosemittels nach Anspruch 4 oder einem der vorhergehenden, dadurch gekennzeichnet, daß diese aus einer Ampulle besteht, welche das Diagnosemittel als Flüssigkeit in der für einen Einmaltest erforderlichen Menge enthält und deren zugeschmolzener Halsbereich derart mit einer Sollbruchstelle versehen ist, daß nach Abbrechen des über die Sollbruchstelle vorragenden Teils des Halsbereiches im Bereich des verbleibenden Teils der Sollbruchstelle eine scharfkantige Schneide unter gleichzeitiger Öffnung des Halsbereiches vorhanden ist.

8. Vorrichtung zur Verwendung eines Diagnosemittels nach Anspruch 1 oder einem der vorhergehenden, dadurch gekennzeichnet, daß diese eine Ampulle (1) aufweist, welche das Diagnosemittel (2) als Flüssigkeit in der für einen Einmaltest erforderlichen Menge enthält und einendig mittels eines Stopfens (3) od.dgl. dicht verschlossen ist, dessen dem Diagnosemittel (2) zugewandte Stirnfläche (4) mit einer oder mehreren nadelartigen und/oder scharfkantigen Vorsprüngen (5) ausgerüstet ist.

9. Vorrichtung nach Anspruch 8, dadurch gekennzeichnet, daß das der Ampulle (1) abgewandte Ende des Stopfens (3) oder dergleichen als Handhabe ausgeführt ist.

Fig.1

Fig.2